Europäisches Patentamt

European Patent Office

Office européen des brevets

(11) Publication number: **0 449 505 B1**

## EUROPEAN PATENT SPECIFICATION

(45) Date of publication of patent specification: 24.05.95 (51) Int. Cl.⁶: **C08F 20/54**, C07D 311/88, C07C 233/09, C09K 11/06

(21) Application number: 91302466.7

(22) Date of filing: 21.03.91

(54) **Fluorescent polymers and ingredients therefor.**

(30) Priority: 26.03.90 US 498657
04.09.90 US 576880
13.09.90 US 581937

(43) Date of publication of application:
**02.10.91 Bulletin 91/40**

(45) Publication of the grant of the patent:
**24.05.95 Bulletin 95/21**

(84) Designated Contracting States:
**DE ES FR GB IT NL**

(56) References cited:

DATABASE BEILSTIEN, no. 212682
(HOST:STN), DE; & J. AM. PHARM. ASSOC.,
43, pages 32-33, 1954

POLYMER BULLETIN, vol. 2, 1980, pages
51-56, Springer-Verlag; C.I. SIMIONESCU et
al.: "New fluorene containing monomers and
polymers"

JOURNAL OF POLYMER SCIENCE, POLYMER
CHEMISTRY EDITION, vol. 20, 1982, pages
2727-2730, John Wiley & Sons, Inc.; T. OISHI
et al.: "Syntheses and polymerizations of

some N-fluorenyl acrylamide and
methacrylamide"

(73) Proprietor: **NALCO CHEMICAL COMPANY**
**One Nalco Center**
**Naperville**
**Illinois 60563-1198 (US)**

(72) Inventor: **Fong, Dodd Wing**
**1275 Leverenz Road**
**Naperville,**
**Illinois 60564 (US)**

(74) Representative: **Harrison, David Christopher**
**et al**
**MEWBURN ELLIS**
**York House**
**23 Kingsway**
**London WC2B 6HP (GB)**

**Description**

This invention relates to a method of making fluorescent polymers and to monomer ingredients therefor.

It has been known that acrylamide polymers can be modified in aqueous solution with organic dye molecules which can form stable carbo-cations reversibly in water (US-A-4,813,973). An example of a suitable dye is dibenzosuberenol. Polymers were tagged by mixing the dye with polyacrylamide in a dilute aqueous solution with an acid catalyst at room temperature for 10 to 24 hours. The polymers were recovered by precipitation in methanol and analyzed spectrophotometrically. The process requires the dissolution of the polymer to be tagged in water and the polymer to be acrylamide based.

The modification of polyacrylamide in dilute aqueous or mixed solvent system is not very practical. Copolymerization with a fluorescent monomer would be a better alternative to prepare tagged polymers especially for systems like high molecular weight cationic polymers.

We have found that vinyl polymers (not necessarily acrylamide based) can readily be prepared by addition polymerization of a vinyl monomer with a fluorescent acrylamide monomer, N-dibenzosuberenylacrylamide (N-5-(5H-dibenzol[a,d]cycloheptenyl)acrylamide). This process is more economical and practical for commercial use than the process described in US-A-4,813,973.

The method is preferably such that the water soluble polymer contains from 0.01 - 2.0 mole % especially from 0.1 - 2.0 mole % of N-dibenzosuberenylacrylamide and, optionally, vinyl monomer polymerized with N-9-xanthenylacrylamide. In a preferred embodiment, the water soluble vinyl monomer that is polymerized with N-dibenzosuberenylacrylamide and optionally N-9-xanthenylacrylamide is acrylamide or acrylic acid monomer or mixtures thereof.

Water soluble cationic monomers can be modified in accordance with the teachings of the invention and are illustrated by such monomers as diallyldimethyl ammonium chloride and dimethylaminoethyl acrylate and diethyldimethylaminopropylmethacrylate.

The amount of N-dibenzosuberenylacrylamide that may be co-polymerized with the monomers described above will vary from as little as O.O1 up to 2 mole %. Since the acrylamide monomers are water soluble the amount used in preparing water soluble polymers should not be such as to reduce the solubility of the product sought to be tagged.

PREPARATION OF THE ACRYLAMIDE MONOMERS

The reaction media may be water, acetic acid, formic acid, or in water-acetic acid or water-formic acid mixtures. Preferred solvents contain from 5% to 90% acetic or formic acids. The reaction temperature is from 0°C to 40°C, preferably 10°C to 30°C.

In the examples below, the acrylamide used was an aqueous 40% solution.

For the preparation of N-9-xanthenylacrylamide see e.g. Bond et al J. Am. Pharm. Assoc <u>43</u> (1954) 32, 33. This group shows little fluorescence.

EXAMPLE 1

Preparation of N-dibenzosuberenylacrylamide

REAGENTS:

| | |
|---|---|
| Acetic Acid | 50 g |
| Acrylamide | 10 g (0.141 mole) |
| Dibenzosuberenol | 2.04 g (0.010 mole) |

REACTION CONDITIONS:

Dibenzosuberenol was added with stirring into an acetic acid solution of acrylamide at room temperature.

RESULTS:

A clear yellow solution was formed. After stirring at room temperature for 24 hours, a white solid separated from solution. The solid isolated was washed with water and methanol.

The IR and C13 NMR spectra of this sample is consistent with the anticipated N-dibenzosuberenylacrylamide.

EXAMPLE 2

Batch polymerization of acrylic acid and N-dibenzosuberenylacrylamide.

REAGENTS:

A
```
Acrylic Acid                                50.75 g

27.2% solution of
N-dibenzosuberenyl-acrylamide
in trifluoroacetic acid               3.73 g (2 wt% (0.6
                                                 mole % of
                                                 monomer)

H₂O, DI                                     54.97 g
```

B
```
Ammonium persulfate                    0.89 g

H₂O, DI                                    16    g
```

C
```
Sodium bisulfite                        2.66 g

H₂O, DI                                    16    g
```

Monomer solution A was charged into a 300-ml Parr reactor. The solution was heated to 35°C and initiator solutions B and C were added into the monomer solution with stirring. The valves of the reactor were quickly closed. The reaction mixture was warmed up slowly and an exothermic reaction occurred at about 50°C and the temperature rose quickly to 135°C. After the reaction was completed, the polymer was characterized by GPC using UV and fluorescence detectors. Results show the polymer was fluorescent.

EXAMPLE 3

Batch polymerization of acrylic acid, acrylamide and N-dibenzosuberenylacrylamide.

REAGENTS:

|   |   |   |   |
|---|---|---|---|
| A | Acrylic Acid | 25.55 g | |
|   | Acrylamide (48.4% solution) | 52.07 g | |
|   | 50% NaOH | 12.64 g | |
|   | 27.2% Soln of N-dibenzosuberenyl-acrylamide in trifluoroacetic acid | 3.23 g | (1.7 wt %) (0.5 mole %) of monomers) |
|   | pH of the monomer solution = 5.0 | | |
|   | H₂O, DI | 19.19 g | |
| B | Ammonium Persulfate | 0.80 g | |
|   | H₂O, DI | 16 g | |
| C | Sodium bisulfite | 2.66 g | |
|   | H₂O, DI | 16 g | |

Monomer solution A was charged into a 300-ml Parr reactor. The solution was heated to 35°C and initiator solutions B and C were added into the monomer solution with stirring. The valves of the reactor were quickly closed. The reaction was highly exothermic and the temperature of reaction mixture rose quickly to about 140°C. After the reaction was completed the polymer was characterized by GPC using UV and fluorescence detectors. Results show the polymer was fluorescent.

Summary:

N-dibenzosuberenylacrylamide monomer has been prepared and characterized by NMR and IR.
The N-dibenzosuberenylacrylamide group shows strong fluorescence at 345 nm (ex 295 nm). Polyacrylic acid and poly(acrylic acid-acrylamide) prepared with 2 wt. % of N-dibenzosuberenylacrylamide comonomer show fluorescence at 345 nm and can be detected readily with a fluorometer.

**Claims**

1. A method of making fluorescent polymer which consists of the addition - polymerization of a vinyl monomer with N-dibenzosuberenylacrylamide (systematic name: N-5-(5H-dibenzo[a,d]cycloheptenyl)-acrylamide).

2. A method according to claim 1 wherein the polymer is water-soluble and contains from 0.1 - 2.0 mole % of N-dibenzosuberenylacrylamide.

3. A method according to claim 1 or claim 2 wherein the vinyl monomer is acrylic acid or acrylamide or a mixture thereof.

4. A method according to any one of the preceding claims wherein the or a vinyl monomer of the vinyl polymer is diallyldimethyl ammonium chloride, dimethyl-aminoethyl acrylate or diethyldimethyl-amino propylmethacrylate.

5. A method according to any one of the preceding claims wherein the vinyl monomer is also polymerized with N-9-xanthenylacrylamide.

6. N-dibenzosuberenylacrylamide (systematic name: N-5-(5H-dibenzo[a,d]cycloheptenyl)acrylamide).

7. Use of N-dibenzosuberenylacrylamide in the formation of a fluorescent vinyl polymer.

**Patentansprüche**

1. Verfahren zur Herstellung eines fluoreszierenden Polymers, bestehend in der Additionspolymerisation eines Vinylmonomers mit N-Dibenzosuberenylacrylamid (Systemname: N-5-(5H-Dibenzo[a,d]-cycloheptenyl)acrylamid).

2. Verfahren nach Anspruch 1, worin das Polymer wasserlöslich ist und 0,1 - 2,0 Mol-% N-Dibenzosuberenylacrylamid enthält.

3. Verfahren nach Anspruch 1 oder 2, worin das Vinylmonomer Acrylsäure oder Acrylamid oder ein Gemisch davon ist.

4. Verfahren nach irgendeinem der vorangegangenen Ansprüche, worin das oder ein Vinylmonomer des Vinylpolymers Diallyldimethylammoniumchlorid, Dimethylaminoethylacrylat oder Diethyldimethylaminopropylmethacrylat ist.

5. Verfahren nach irgendeinem der vorangegangenen Ansprüche, worin das Vinylmonomer auch mit N-9-Xanthenylacrylamid polymerisiert wird.

6. N-Dibenzosuberenylacrylamid (Systemname: N-5-(5H-Dibenzo[a,d]cycloheptenyl)acrylamid).

7. Verwendung von N-Dibenzosuberenylacrylamid zur Bildung eines fluoreszierenden Vinylpolymers.

**Revendications**

1. Méthode pour fabriquer un polymère fluorescent qui consiste en la polymérisation par addition d'un monomère vinylique avec du N-dibenzosubérénylacrylamide (nom systématique: N-5-(5H-dibenzo[a,d]-cycloheptényl)acrylamide).

2. Méthode selon la revendication 1 dans laquelle le polymère est soluble dans l'eau et contient de 0,1 à 2,0 moles % de N-dibenzosubérénylacrylamide.

3. Méthode selon la revendication 1 ou la revendication 2 dans laquelle le monomère vinylique est un acide acrylique ou un acrylamide ou un mélange de ces derniers.

4. Méthode selon l'une quelconque des revendications précédentes dans laquelle le ou un monomère vinylique du polymère vinylique est un chlorure de diallyldiméthyl ammonium, un diméthyl-aminoéthyl acrylate ou un diéthyldiméthyl-amino propylméthacrylate.

5. Méthode selon l'une quelconque des revendications précédentes dans laquelle le monomère vinylique est également polymérisé avec du N-9-xanthénylacrylamide.

6. N-dibenzosubérénylacrylamide (nom systématique : N-5-(5H-dibenzo[a,d]cyclohepténýl)acrylamide).

7. Utilisation du N-dibenzosubérénylacrylamide dans la formation d'un polymère vinylique fluorescent.